# EUROPEAN PATENT APPLICATION

(11) **EP 2 891 652 A1**
(43) Date of publication of application: **08.07.2015**
(21) Application number: 13832335.7
(22) Date of filing: 27.08.2013
(51) Int. Cl.: C07D 303/16, C07D 301/03, C07D 301/14

(54) **ALICYCLIC EPOXY COMPOUND AND METHOD FOR PRODUCING SAME**

(30) Priority: 30.08.2012 JP 2012189454
(71) Applicant: Daicel Corporation, Osaka-shi, Osaka 530-0001 (JP)
(72) Inventor: NAKAMURA, Ryota, Ohtake-shi Hiroshima 739-0695 (JP); WATANABE, Jun, Ohtake-shi Hiroshima 739-0695 (JP)
(74) Representative: Grünecker Patent- und Rechtsanwälte PartG mbB
(86) International application number: PCT/JP2013/072795
(87) International publication number: WO 2014/034628

(57) **Abstract**

Provided is a novel epoxide that is highly soluble in organic solvents, is lowly volatile, contains a highly reactive epoxy group, and still resists polymerization by itself.

The cycloaliphatic epoxide according to the present invention is a cycloaliphatic epoxide represented by Formula (1) : 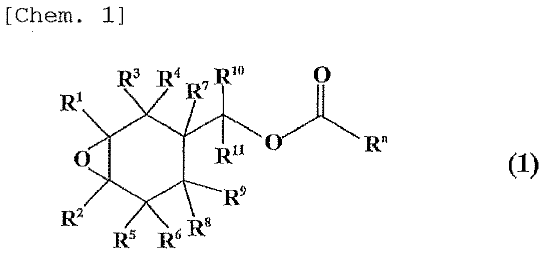 where R¹ to R¹¹ are each independently selected from hydrogen and C₁-C₄ straight or branched chain alkyl; and R^{a} represents C₈-C₂₃ branched chain alkyl. R^{a} is preferably C₈-C₁₇ branched chain alkyl.

## Description

### Technical Field

The present invention relates to cycloaliphatic epoxides (alicyclic epoxy compounds) and methods for producing the same. More specifically, the present invention relates to a cycloaliphatic epoxide that is highly soluble in organic solvents, is lowly volatile, contains a highly reactive epoxy group, and still resists polymerization by itself.

### Background Art

Epoxides contain one or more epoxy groups per molecule (in one molecule) and serve a variety of uses. The epoxides are mainly used as principal components to form epoxy resins that are used typically in a variety of structural materials. Their uses, however, have gone beyond this and become wider and wider recently. For example, some epoxides are used as additives to impart a specific function or functions to materials. The epoxides are used as additives typically as a reactive diluent to allow an epoxy resin to have a lower viscosity (see, for example, Patent Literature (PTL) 1); and as a stabilizer (acid scavenger) to scavenge an acid formed in a poly(vinylidene chloride) resin (see, for example, PTL 2).

The epoxides to be used as the additives require low volatility. The low volatility is such a property as to resist volatilization even upon heating typically in curing of the epoxy resins or melt molding of the resins. To exhibit functions as a reactive diluent or a stabilizer (acid scavenger) sufficiently, the epoxides further require high reactivity with an epoxy group or an acid and resistance to polymerization by themselves. Assume that an epoxide is mixed with a resin each as a solution in an organic solvent so as to uniformly disperse in the resin, where the resin is exemplified by an epoxy resin and a poly(vinylidene chloride). In this case, the epoxide requires good solubility in various organic solvents so as to perform the mixing efficiently.

### Citation List

### Patent Literature

PTL 1: Japanese Unexamined Patent Application Publication (JP-A) No. H11-80317
PTL 2: JP-A No. 2003-026882

### Summary of Invention

### Technical Problem

However, under present circumstances, there has been obtained no epoxide that has the required properties in good balance at high levels.

Accordingly, it is an object of the present invention to provide a novel epoxide that is highly soluble in organic solvents, is lowly volatile, contains a highly reactive epoxy group, and still resists polymerization by itself. Solution to Problem

The present inventors have found a cycloaliphatic epoxide having a specific structure and have found that the cycloaliphatic epoxide is highly soluble in organic solvents, is lowly volatile, contains a highly reactive epoxy group, and still resists polymerization by itself. The present invention has been made based on these findings.

Specifically, the present invention provides, in one aspect, a cycloaliphatic epoxide represented by Formula (1): 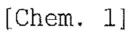 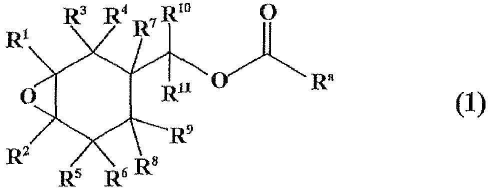 where R¹ to R¹¹ are each independently selected from hydrogen and C₁-C₄ straight or branched chain alkyl; and R^{a} represents C₈-C₂₃ branched chain alkyl.

In the cycloaliphatic epoxide, R^{a} is preferably C₈-C₁₇ branched chain alkyl.

In the cycloaliphatic epoxide, R¹ to R¹¹ are each preferably hydrogen.

In another aspect, the present invention provides a method for producing a cycloaliphatic epoxide. The method includes the step A of allowing a compound represented by Formula (2) to react with an oxidant to form a cycloaliphatic epoxide represented by Formula (1) as the above-mentioned cycloaliphatic epoxide. Formulae (2) and (1) are expressed as follows: 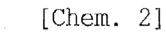 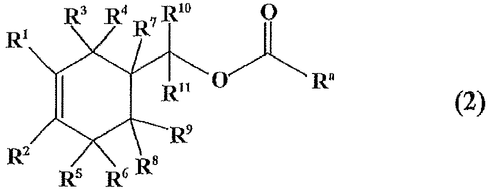 where R¹ to R¹¹ are each independently selected from hydrogen and C₁-C₄ straight or branched chain alkyl; and R^{a} represents C₈-C₂₃ branched chain alkyl, 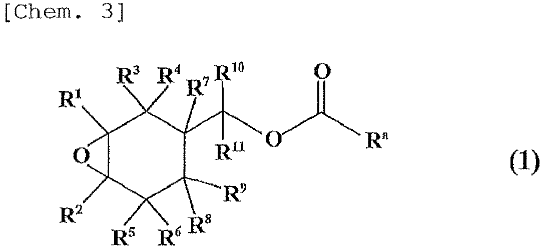 where R¹ to R¹¹ and R^{a} are as defined above.

The method for producing a cycloaliphatic epoxide may further include, before the step A, the step B of allowing a compound represented by Formula (3) to react with a compound represented by Formula (4) or a derivative thereof to form the compound represented by Formula (2). Formulae (3) and (4) are expressed as follows: 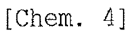 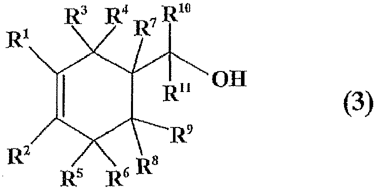 where R¹ to R¹¹ are as defined above, 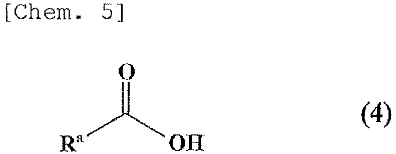 where R^{a} is as defined above.

### Advantageous Effects of Invention

The cycloaliphatic epoxide according to the present invention, as having the structure, is highly soluble in organic solvents, is lowly volatile, contains a highly reactive epoxy group, and still resists polymerization by itself. The cycloaliphatic epoxide according to the present invention is therefore particularly preferably usable typically as reactive diluents and stabilizers (acid scavengers).

### Brief Description of Drawings

[Fig. 1] Fig. 1 depicts a gas chromatogram (upper chart) and a mass spectrum (lower chart) of a peak at a retention time of 26.55 minutes, in GC-MS of 3,4-epoxycyclohexylmethyl 3,5,5-trimethylhexanoate prepared in Example 1.
[Fig. 2] Fig. 2 depicts a gas chromatogram (upper chart) and a mass spectrum (lower chart) of a peak at a retention time of 24.27 minutes, in GC-MS of 3-cyclohexenylmethyl 3,5,5-trimethylhexanoate.
[Fig. 3] Fig. 3 is a ¹H-NMR spectrum of 3,4-epoxycyclohexylmethyl 3,5,5-trimethylhexanoate prepared in Example 1.
[Fig. 4] Fig. 4 is a ¹H-NMR spectrum of 3-cyclohexenylmethyl 3,5,5-trimethylhexanoate.
[Fig. 5] Fig. 5 is an IR spectrum of 3,4-epoxycyclohexylmethyl 3,5,5-trimethylhexanoate prepared in Example 1.
[Fig. 6] Fig. 6 is an IR spectrum of 3-cyclohexenylmethyl 3,5,5-trimethylhexanoate.

### Description of Embodiments

### Cycloaliphatic Epoxide

The cycloaliphatic epoxide according to the present invention is a compound represented by Formula (1):

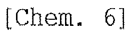

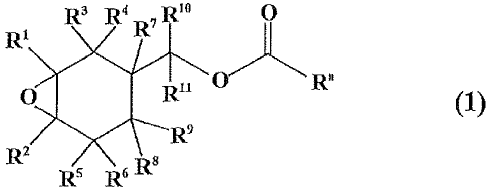

In Formula (1), R¹ to R¹¹ (R¹, R², R³, R⁴, R⁵, R⁶, R⁷, R⁸, R⁹, R¹⁰, and R¹¹) are each independently selected from hydrogen and C₁-C₄ straight or branched chain alkyl. Specifically, R¹ to R¹¹ may be identical to or different from one another. The C₁-C₄ straight or branched chain alkyl is exemplified by methyl, ethyl, propyl, isopropyl, butyl, isobutyl, s-butyl, and t-butyl. Among them, R¹ to R¹¹ in Formula (1) are each preferably selected from hydrogen and methyl and are more preferably each hydrogen.

In Formula (1), R^{a} represents C₈-C₂₃ branched chain alkyl. The C₈-C₂₃ branched chain alkyl is not limited, as long as being alkyl including a branched-structure carbon chain and containing 8 to 23 carbon atoms that constitute the carbon chain. Such C₈-C₂₃ branched chain alkyl is exemplified by methylheptyl, dimethylhexyl, ethylhexyl, trimethylpentyl, ethylmethylpentyl, propylpentyl, tetramethylbutyl, ethyldimethylbutyl, diethylbutyl, methyloctyl, dimethylheptyl, trimethylhexyl, tetramethylpentyl, pentamethylbutyl, ethylheptyl, ethylmethylhexyl, ethyldimethylpentyl, hexylnonyl, heptyldecyl, dimethylpentadecyl, hexamethylundecyl, and dimethylheptadecyl. The above-exemplified C₈-C₂₃ branched chain alkyl includes structural isomers. Among them, preferred is C₈-C₁₇ branched chain alkyl for excellent solubility in organic solvents (in particular, in aliphatic organic solvents containing an aliphatic hydrocarbon chain); more preferred is C₈ branched chain alkyl for excellent availability of a corresponding starting material and satisfactory productivity; and particularly preferred is trimethylpentyl.

Specifically, the cycloaliphatic epoxide according to the present invention is exemplified by compounds represented by Formulae (1-1) to (1-59):

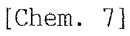

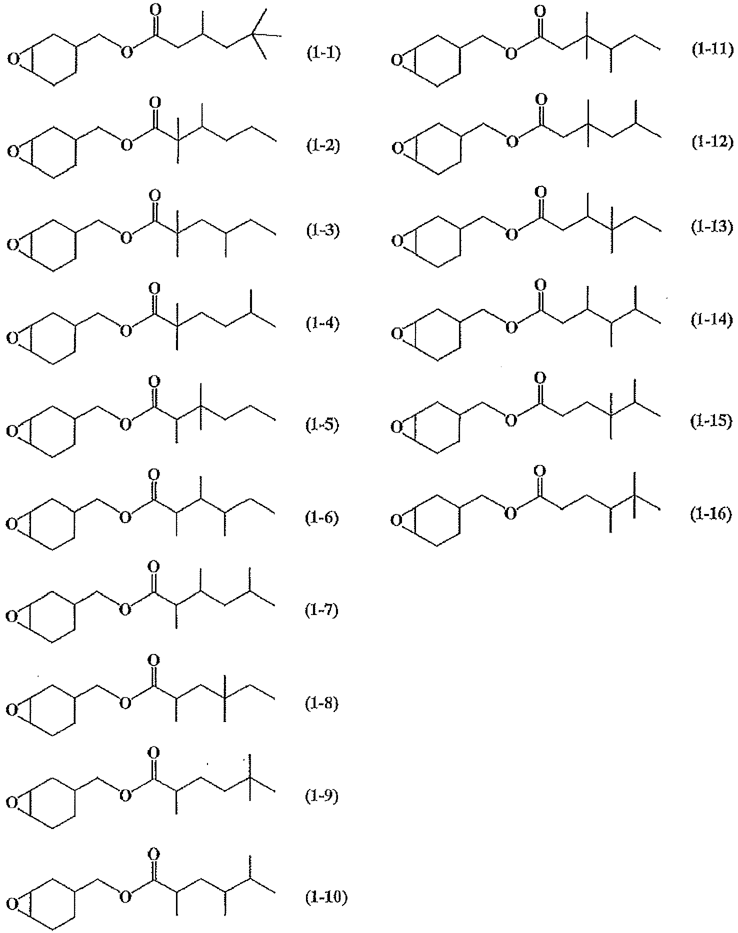

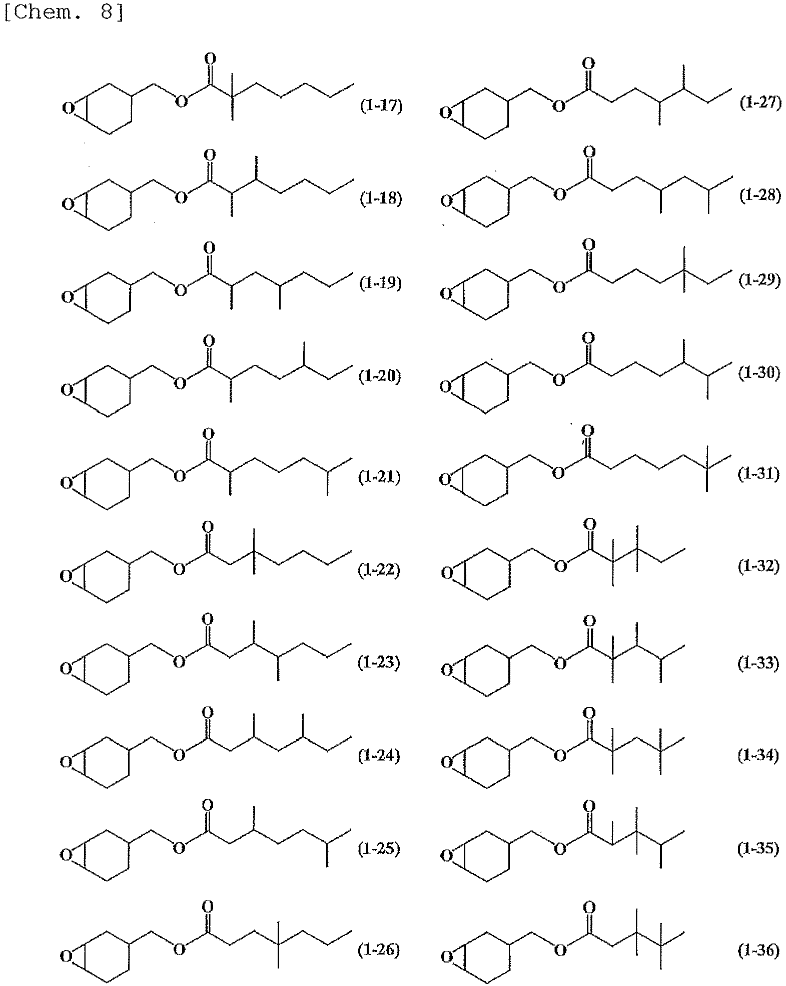

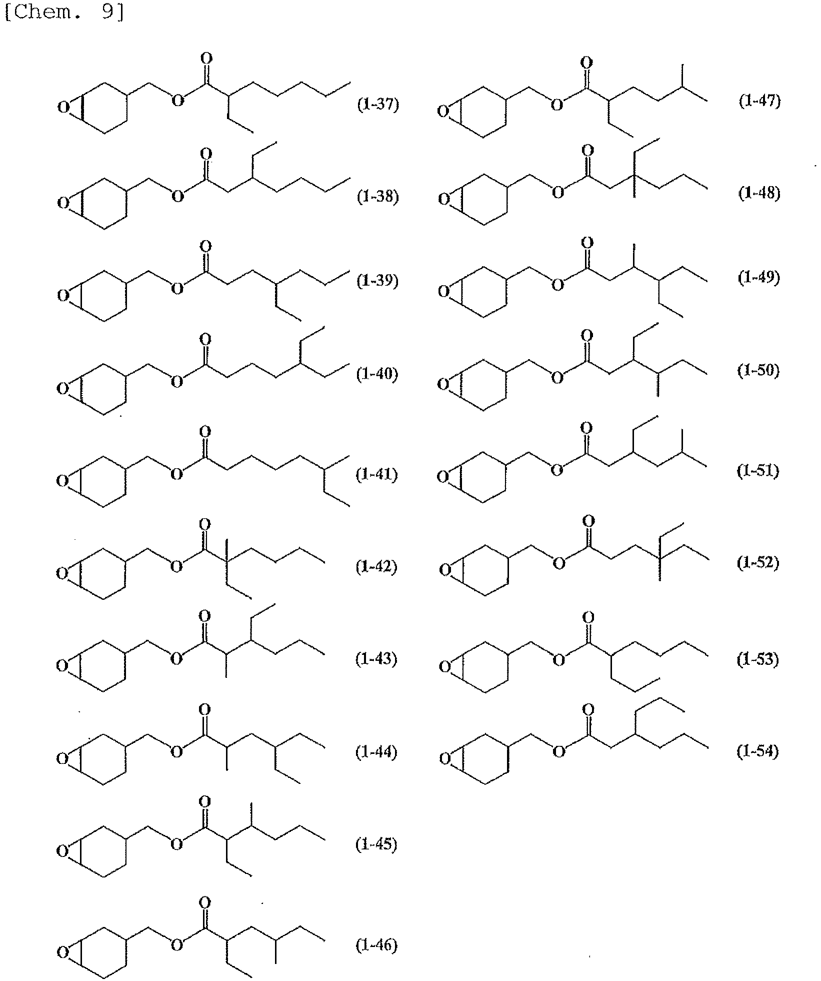

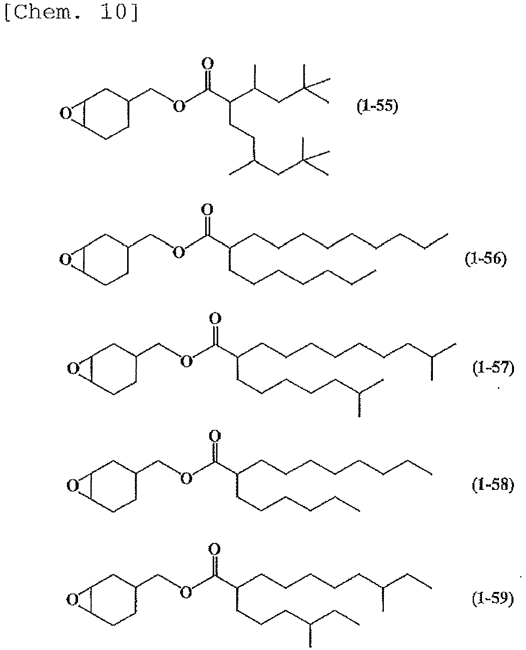

As is indicated in Formula (1), the cycloaliphatic epoxide according to the present invention contains a cyclohexene oxide group as a reactive functional group. The "cyclohexene oxide group" refers to an epoxy group including an oxygen atom and two carbon atoms defining a cyclic structure of cyclohexane ring. The cycloaliphatic epoxide is particularly highly reactive with acids and compounds each containing active hydrogen. The compounds containing active hydrogen are exemplified by alcohols, mercaptans, and carboxylic acids. The cycloaliphatic epoxide contains the epoxy group in a number of one alone per molecule (in one molecule) and more resists polymerization by itself as compared with compounds containing two or more epoxy groups per molecule. The cycloaliphatic epoxide according to the present invention includes the structure represented by Formula (1). In particular, the cycloaliphatic epoxide contains the branched chain alkyl containing 8 or more carbon atoms. This allows the cycloaliphatic epoxide to be lowly volatile and highly soluble in various organic solvents (in particular, aliphatic organic solvents containing an aliphatic hydrocarbon chain).

### Cycloaliphatic Epoxide Production Method

The cycloaliphatic epoxide according to the present invention (the compound represented by Formula (1)) may be produced typically, but not limitatively, by a method including a step A of allowing a compound represented by Formula (2) to react with an oxidant to form the cycloaliphatic epoxide represented by Formula (1). The "step A" herein refers to the step of allowing a compound represented by Formula (2) to react with an oxidant to form the cycloaliphatic epoxide represented by Formula (1). Formula (2) is expressed as follows: 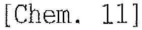 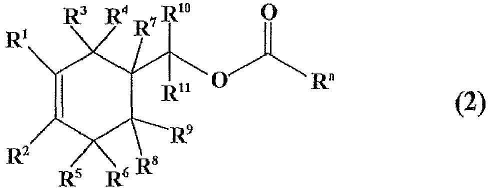 where R¹ to R¹¹ and R^{a} are as defined above.

The oxidant (oxidizer) in the step A is used as an epoxidizing agent to epoxidize a carbon-carbon double bond of the compound represented by Formula (2). The carbon-carbon double bond is the carbon-carbon double bond in the cyclohexene ring. The oxidant may be selected from among known oxidants for use in epoxidation of carbon-carbon double bond and is exemplified by, but not limited to, organic peroxy acids (e.g., performic acid, peracetic acid, trifluoroperacetic acid, perbenzoic acid, meta-chloroperbenzoic acid, and monoperoxyphthalic acid), inorganic peroxy acids (e.g., permanganic acid), hydrogen peroxide, peroxides, hydroperoxides, peroxoacids, peroxoacid salts, and other peroxides. Among them, organic peroxycarboxylic acids containing approximately no water are preferred so as to give a compound having a high degree of epoxidation. Specifically, preferred are organic peroxycarboxylic acids having a moisture content (water content) of preferably 0.8 percent by weight or less, and more preferably 0.6 percent by weight or less. The organic peroxycarboxylic acids containing approximately no water may be produced by oxidation of an aldehyde (e.g., acetaldehyde) with air. Typically, peracetic acid of this type is produced by a method described in German Patent Laid-Open (DE-A1) No. 1418465 and JP-A No. S54-3006. The method enables continuous synthetic production of a high-concentration organic peroxycarboxylic acid in a large amount and can give the organic peroxycarboxylic acid substantially inexpensively, as compared with production of the organic peroxycarboxylic acid by synthetically preparing the organic peroxycarboxylic acid from hydrogen peroxide and extracting the target compound with a solvent.

The oxidant may be used in an amount not critical, but preferably from 1.0 to 5.0 moles, more preferably from 1.05 to 3.0 moles, and furthermore preferably from 1.1 to 2.0 moles, per mole of the compound represented by Formula (2). The oxidant, if used in an amount of less than 1.0 mole, may fail to provide the cycloaliphatic epoxide represented by Formula (1) in a high yield. In contrast, the oxidant, if used in an amount of greater than 5.0 moles, may readily cause side reactions or may invite economical disadvantages.

The reaction between the compound represented by Formula (2) and the oxidant may be performed in the presence of, or in the absence of, an organic solvent. The organic solvent is exemplified by alcohols such as t-butyl alcohol; aliphatic hydrocarbons such as hexane, heptane, and octane; alicyclic hydrocarbons such as cyclohexane; aromatic hydrocarbons such as benzene, toluene, xylenes, and ethylbenzene; halogenated hydrocarbons such as chloroform, methylene chloride, and 1,2-dichloroethane; esters such as ethyl acetate; amides such as N,N-dimethylformamide and N,N-dimethylacetamide; nitriles such as acetonitrile, propionitrile, and benzonitrile; and organic acids such as acetic acid. Each of different organic solvents may be used alone or in combination as the organic solvent. Of the organic solvents, ester organic solvents (esters) are preferred, of which ethyl acetate is more preferred.

The organic solvent may be used in an amount not critical, but preferably from 50 to 1000 parts by weight, and more preferably from 100 to 500 parts by weight, per 100 parts by weight of the compound represented by Formula (2).

The reaction between the compound represented by Formula (2) and the oxidant may be performed at a temperature (reaction temperature) not critical, but preferably from 0°C to 100°C, more preferably from 10°C to 80°C, and furthermore preferably from 20°C to 70°C, although the temperature may vary depending on the type of the oxidant to be used. The reaction, if performed at a temperature of lower than 0°C, may proceed slowly to cause lower productivity. In contrast, the reaction, if performed at a temperature of higher than 100°C, may cause the oxidant to decompose and/or may cause side reactions to occur frequently. This may cause the target epoxide to produce in a lower yield with lower productivity.

The reaction between the compound represented by Formula (2) and the oxidant may be performed for a time (reaction time) not critical, but preferably from 1 to 20 hours, more preferably from 1.5 to 15 hours, and furthermore preferably from 2 to 8 hours, although the reaction time may vary depending typically on the type of the oxidant to be used and the reaction temperature. The reaction, if performed for a time shorter than one hour, may fail to proceed sufficiently. In contrast, the reaction, if performed for a time of longer than 20 hours, may cause the oxidant to decompose and/or may cause side reactions to occur frequently. This may cause the target epoxide to produce in a lower yield with lower productivity.

The reaction between the compound represented by Formula (2) and the oxidant may be performed at normal atmospheric pressure, under reduced pressure, or under pressure (under a load). The reaction may be performed in any atmosphere without limitation, as long as not being adversely affected. The atmosphere may be selected typically from air, nitrogen, and argon atmospheres. The reaction may be performed in any system such as batch, semi-batch, and continuous systems.

The reaction between the compound represented by Formula (2) and the oxidant may be completed typically by the addition of sodium thiosulfate or sodium sulfite.

After the completion of the reaction, the cycloaliphatic epoxide represented by Formula (1) as a reaction product may be separated/purified by a separation method such as filtration, concentration, distillation, extraction, crystallization, recrystallization, or column chromatography, or a separation method as any combination of them.

The method for producing the cycloaliphatic epoxide according to the present invention may further include a step B before the step A. The step B is the step of preparing the compound represented by Formula (2). The step B is exemplified by the step of allowing a compound represented by Formula (3) to react with a compound represented by Formula (4) or a derivative thereof to form the compound represented by Formula (2). Formulae (3) and (4) are expressed as follows:

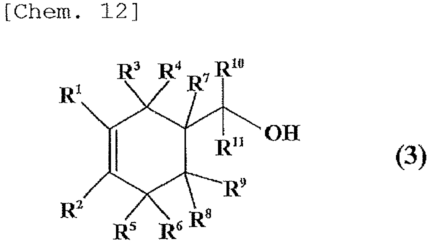

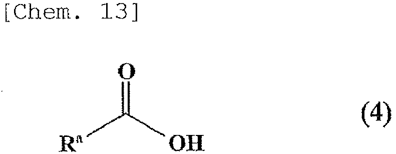

In Formula (3), R¹ to R¹¹ are each independently selected from hydrogen and C₁-C₄ straight or branched chain alkyl. The C₁-C₄ straight or branched chain alkyl is exemplified as with R¹ to R¹¹ in Formula (1).

Specifically, the compound represented by Formula (3) is exemplified by tetrahydrobenzyl alcohol (3-cyclohexene-1-methanol), 1-methyl-3-cyclohexene-1-methanol, 2-methyl-3-cyclohexene-1-methanol, 3-methyl-3-cyclohexene-1-methanol, 4-methyl-3-cyclohexene-1-methanol, 5-methyl-3-cyclohexene-1-methanol, 6-methyl-3-cyclohexene-1-methanol, 1-(3-cyclohexen-1-yl)ethanol, 2-(3-cyclohexen-1-yl)-2-propanol, and 2-[4-methyl-3-cyclohexen-1-yl]-2-propanol.

In Formula (4), R^{a} represents C₈-C₂₃ branched chain alkyl. The C₈-C₂₃ branched chain alkyl is exemplified as with R^{a} in Formula (1).

Specifically, the compound represented by Formula (4) is exemplified by trimethylhexanoic acids (e.g., 3,5,5-trimethylhexanoic acid, 2,2,3-trimethylhexanoic acid, and 2,2,4-trimethylhexanoic acid), dimethylheptanoic acids (e.g., 2,2-dimethylheptanoic acid, 2,3-dimethylheptanoic acid, and 2,4-dimethylheptanoic acid), tetramethylbutanoic acids (e.g., 2,2,3,3-tetramethylbutanoic acid and 2,2,3,4-tetramethylbutanoic acid), ethylheptanoic acids (e.g., 2-ethylheptanoic acid and 3-ethylheptanoic acid), ethylmethylhexanoic acids (e.g., 2-methyl-3-ethylhexanoic acid), pentylhexanoic acids (e.g., 2-pentylhexanoic acid), trimethylheptanoic acids (e.g., 2,5,6-trimethylheptanoic acid), trimethyloctanoic acids (e.g., 2,6,7-trimethyloctanoic acid), hexadecanoic acids (e.g., 2-hexyldecanoic acid), octadecanoic acids (e.g., 5,7,7-trimethyl-2-(1,3,3-trimethylbutyl)octanoic acid, 8-methyl-2-(4-methylhexyl)decanoic acid, and 2-hexylundecanoic acid), and icosanoic acids (e.g., 10-ethyl-2-(6-methylheptyl)undecanoic acid).

The derivative (reactive derivative) of the compound represented by Formula (4) is exemplified by acid halides (acyl halides), acid anhydrides, and esters each derived from the compound represented by Formula (4). The acid halides are exemplified by an acid chloride corresponding to the compound of Formula (4), except with chlorine replacing the hydroxy group in Formula (4). The acid anhydrides are exemplified by an acid anhydride corresponding to the compound represented by Formula (4), except for being formed by dehydration condensation of two molecules of the compound. The esters are exemplified by alkyl esters corresponding to the compound of Formula (4), except with straight or branched chain alkyl replacing hydrogen of the hydroxy group in Formula (4).

The reaction between the compound represented by Formula (3) and the compound represented by Formula (4) or a derivative thereof may be performed by any process that is not limited and may be selected as appropriate from known processes for allowing an alcohol to react with a carboxylic acid or a derivative thereof to form an ester. More specifically, the process is exemplified by processes [a], [b], and [c] as follows. In the process [a], the compound represented by Formula (3) is allowed to react with the compound represented by Formula (4) or a derivative thereof (in particular, the compound represented by Formula (4)) in the presence of, or in the absence of, an organic solvent and in the presence of a strong acid. The strong acid is exemplified by hydrochloric acid, sulfuric acid, and p-toluenesulfonic acid. In the process [b], the compound represented by Formula (3) is allowed to react with the compound represented by Formula (4) or a derivative thereof (in particular, an acid halide) in the presence of, or in the absence of, an organic solvent and in the presence of a base as needed. The base is exemplified by triethylamine, pyridine, and 4-dimethylaminopyridine. In the process [c], the compound represented by Formula (3) is allowed to react with the compound represented by Formula (4) or a derivative thereof (in particular, an ester) in the presence of, or in the absence of, an organic solvent and in the presence of a transesterification catalyst. The transesterification catalyst is exemplified by titanium isopropoxide. The organic solvent to be used in the reaction is exemplified by the organic solvents exemplified in the step A.

The reaction in the step B may be performed under conditions that are not critical and may be selected and determined as appropriate from among conditions in known methods for allowing an alcohol to react with a carboxylic acid or a derivative thereof to form an ester compound. For example, in the process [a], the compound represented by Formula (4) or a derivative thereof (in particular, the compound represented by Formula (4)) may be used in an amount not critical, but preferably from 0.5 to 2 moles, and more preferably from 0.8 to 1.5 moles, per mole of the compound represented by Formula (3). The strong acid in the process [a] may be used in an amount not critical, but preferably from 0.00001 to 0.1 mole, and more preferably from 0.0001 to 0.01 mole, per mole of the compound represented by Formula (3). The reaction temperature in the process [a] is not critical, but may be selected as appropriate within the range of from 0°C to 200°C. The reaction time may be adjusted as appropriate and is not critical.

In the process [b], the compound represented by Formula (4) or a derivative thereof (in particular, an acid halide) may be used in an amount not critical, but preferably from 0.5 to 2 moles, and more preferably from 0.8 to 1.5 moles, per mole of the compound represented by Formula (3). The base in the process [b] may be used in an amount not critical, but preferably from 1 to 5 moles, and more preferably from 1 to 3 moles, per mole of the compound represented by Formula (4) or a derivative thereof (in particular, an acid halide). The reaction temperature in the process [b] is not critical, but may be selected as appropriate within the range of typically from -20°C to 50°C. The reaction time may be adjusted as appropriate and is not critical.

In the process [c], the compound represented by Formula (4) or a derivative thereof (in particular, an ester) may be used in an amount not critical, but preferably from 0.5 to 2 moles, and more preferably from 0.8 to 1.5 moles, per mole of the compound represented by Formula (3). The transesterification catalyst in the process [c] may be used in an amount not critical, but preferably from 0.000001 to 0.01 mole, and more preferably from 0.00001 to 0.001 mole, per mole of the compound represented by Formula (3). The reaction temperature in the process [c] is not critical, but may be selected as appropriate within the range of typically from 0°C to 200°C. The reaction time may be adjusted as appropriate and is not critical.

The reaction between the compound represented by Formula (3) and the compound represented by Formula (4) or a derivative thereof may be performed at normal atmospheric pressure, under reduced pressure, or under pressure (under a load). The reaction may be performed in any atmosphere without limitation, as long as not being adversely affected. The atmosphere may be selected typically from air, nitrogen, and argon atmospheres. The reaction may be performed in any system such as batch, semi-batch, and continuous systems.

After the completion of the reaction, the compound represented by Formula (2) as a reaction product may be separated and purified by a separation method such as filtration, concentration, distillation, extraction, crystallization, recrystallization, and column chromatography, or a separation method as any combination of them. The compound represented by Formula (2) prepared as a result of the reaction may also be subjected to the reaction in the step A without separation/purification.

The method for producing a cycloaliphatic epoxide according to the present invention may also include one or more additional steps in addition to the step A and the step B. Such additional steps are exemplified by the step of purifying a starting material or a product.

The cycloaliphatic epoxide according to the present invention is highly soluble in a variety of organic solvents (in particular, aliphatic organic solvents containing an aliphatic hydrocarbon chain), is lowly volatile, contains a highly reactive epoxy group, and still resists polymerization by itself, as is described above. The cycloaliphatic epoxide is therefore preferably usable particularly as a variety of additives such as reactive diluents and stabilizers (acid scavengers). In addition to the use as additives, the cycloaliphatic epoxide according to the present invention is also usable in a variety of uses such as optical semiconductor encapsulants, adhesives, electrical insulating materials, laminated sheets, coatings, inks, coating materials, sealants, resists, composite materials, transparent substrates, transparent sheets, transparent films, optical devices, optical lenses, optical elements, stereolithographic materials, electronic papers, touch-screen panels, solar cell substrates, optical waveguides, light guide plates, and holographic memories. Examples

The present invention will be illustrated in further detail with reference to several examples below. It should be noted, however, that the examples are by no means intended to limit the scope of the present invention.

### Example 1

Materials were prepared as 300.0 g of 3-cyclohexene-1-methanol (Tokyo Chemical Industry Co., Ltd.), 352.6 g of 3,5,5-trimethylhexanoic acid (trade name Isononanoic Acid, supplied by Kyowa Hakko Chemical Co., Ltd.), and 0.7 g of p-toluenesulfonic acid. The materials were raised in temperature up to 120°C with stirring and subjected to a reaction for 12 hours while being gradually raised in temperature from 120°C to 150°C. The reaction mixture was then gradually decompressed to 10 Torr (about 1330 Pa) at 150°C over 3 hours to remove unreacted starting materials from the reaction mixture.

The resulting reaction mixture was returned to room temperature, combined with 562.4 g of ethyl acetate, 1124.9 g of distilled water, and 0.1 g of sodium hydroxide, followed by stirring for 30 minutes to perform alkali washing. The mixture was left stand to be separated into an organic layer and an aqueous layer, from which the aqueous layer was extracted and removed. The organic layer was combined with 1124.9 g of distilled water and stirred for 30 minutes to perform water washing. The resulting mixture was left stand to be separated into an organic layer and an aqueous layer, from which the aqueous layer was extracted and removed. This operation was performed a total of two times. The collected organic layer was transferred into an eggplant type flask, desolvated on an evaporator at 120°C and 10 Torr (about 1330 Pa), and yielded 511.0 g of 3-cyclohexenylmethyl 3,5,5-trimethylhexanoate.

Next, 484.6 g of the above-prepared 3-cyclohexenylmethyl 3,5,5-trimethylhexanoate and 969.1 g of ethyl acetate were, with stirring at 30°C, combined with 554.8 g of a 30 percent by weight solution of peracetic acid in ethyl acetate (moisture content 0.41 percent by weight) added dropwise over 2 hours, followed by stirring for further 5 hours.

After the reaction, the resulting solution was combined with 2008.4 g of distilled water and stirred for 30 minutes to perform water washing. The mixture was left stand to be separated into an organic layer and an aqueous layer, from which the aqueous layer was extracted and removed. This operation was performed a total of four times. Next, the organic layer was desolvated on an evaporator at 150°C and 10 Torr (about 1330 Pa) and yielded 505.0 g of 3,4-epoxycyclohexylmethyl 3,5,5-trimethylhexanoate.

The above-prepared product (3,4-epoxycyclohexylmethyl 3,5,5-trimethylhexanoate) had an epoxy equivalent of 276.3. The product was analyzed by IR, GC-MS, and NMR.

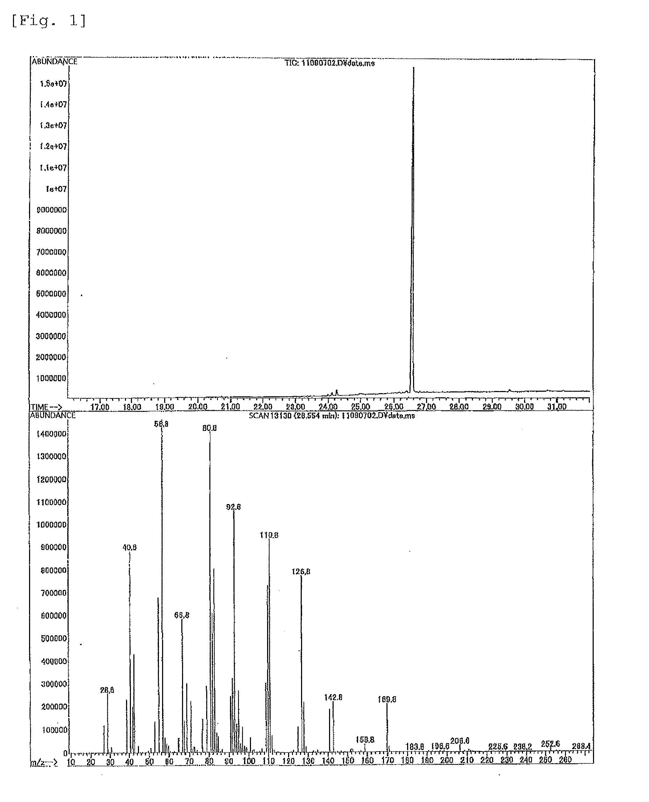

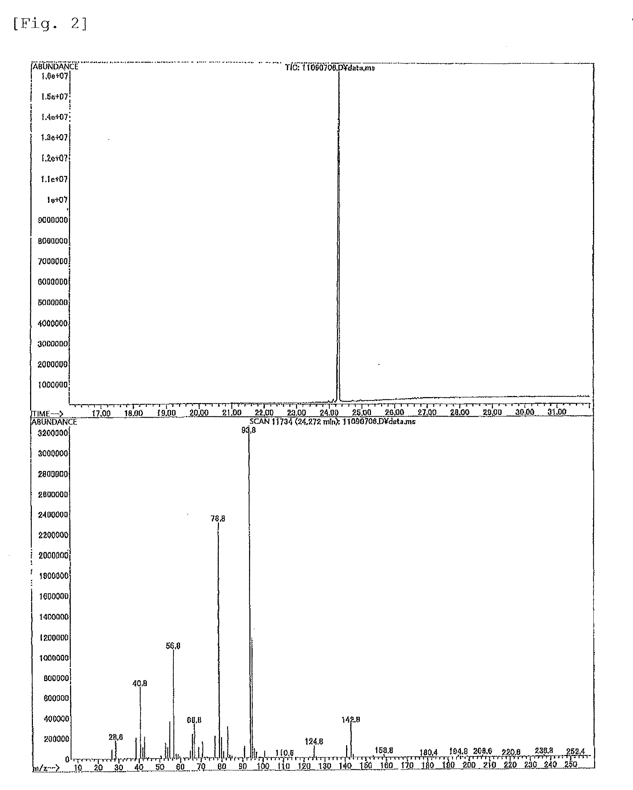

Fig. 1 depicts a gas chromatogram (upper chart) and a mass spectrum (lower chart) of a peak at a retention time of 26.55 minutes, in GC-MS of the product (3,4-epoxycyclohexylmethyl 3,5,5-trimethylhexanoate). Fig. 2 depicts a gas chromatogram (upper chart) and a mass spectrum (lower chart) of a peak at a retention time of 24.27 minutes, in GC-MS of the starting material (3-cyclohexenylmethyl 3,5,5-trimethylhexanoate). As is indicated in the mass spectrum of the product in Fig. 1, peaks (parent ion: 252.4) appearing in Fig. 2 appear in trace amounts and are replaced with peaks (parent ion: 268.4) of the product.

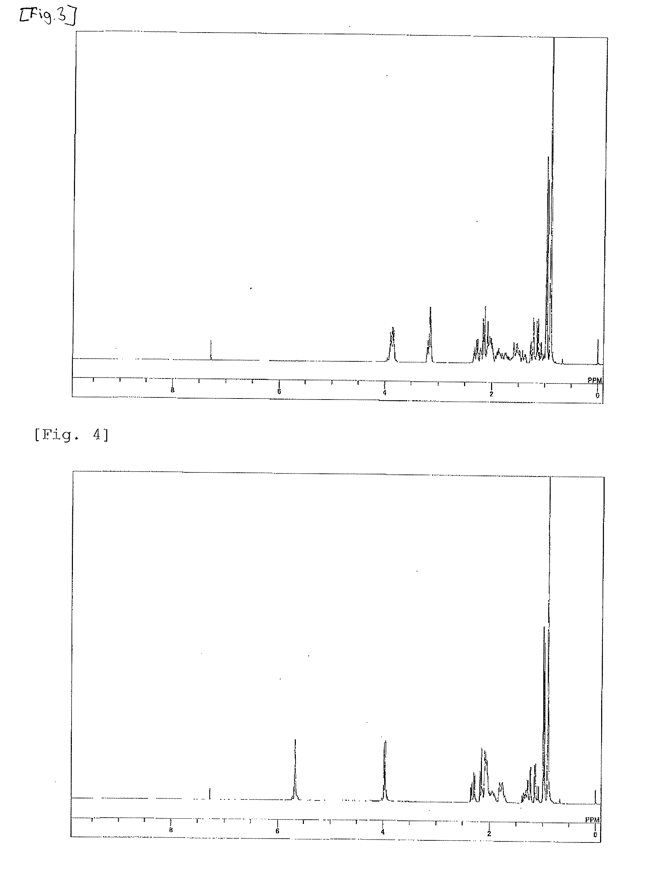

In the ¹H-NMR spectrum measurement of the product (3,4-epoxycyclohexylmethyl 3,5,5-trimethylhexanoate), there were observed peaks as follows (see Fig. 3):
¹H-NMR (270 MHz, CDCl₃, 25°C): δ = 0.91 (s, 9H), 0.98 (d, J = 6.5 MHz, 3H), 1.01-1.28 (m, 3H), 1.37-2.34 (m, 9H), 3.15-3.20 (m, 2H), 3.83-3.91 (m, 2H) ppm.

In the ¹H-NMR spectrum measurement of the starting material (3-cyclohexenylmethyl 3,5,5-trimethylhexanoate), there were observed peaks as follows (see Fig. 4):
¹H-NMR (270 MHz, CDCl₃, 25°C) : δ = 0.91 (s, 9H), 0.99 (d, J = 6.5 MHz, 3H), 1.08-1.39 (m, 3H), 1.71-1.83 (m, 2H), 1.90-2.18 (m, 6H), 2.28-2.35 (m, 1H), 3.91-4.02 (m, 2H), 5.62-5.71 (m, 2H).

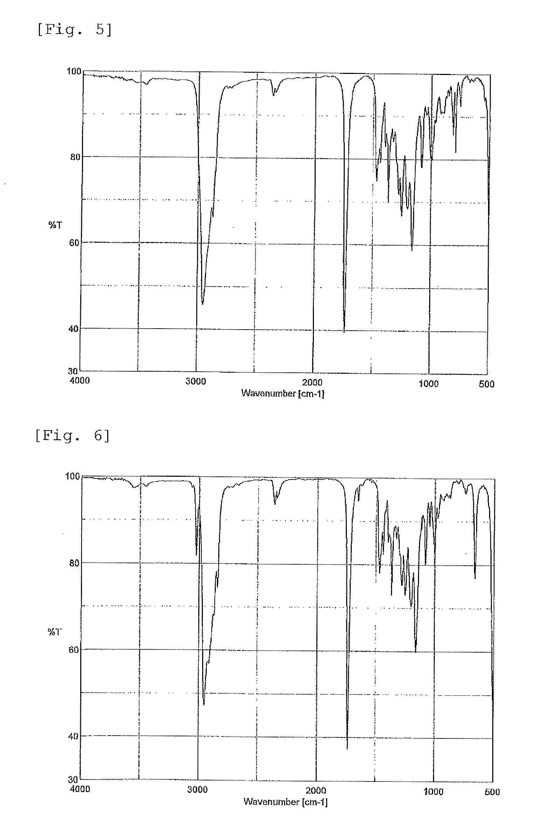

Fig. 5 is the IR spectrum of the product (3,4-epoxycyclohexylmethyl 3,5,5-trimethylhexanoate); and Fig. 6 is the IR spectrum of the starting material (3-cyclohexenylmethyl 3,5,5-trimethylhexanoate). As indicated in Figs. 5 and 6, an absorption of double bond was observed at 1650 cm⁻¹ in the IR spectrum of the starting material (3-cyclohexenylmethyl 3,5,5-trimethylhexanoate); whereas this absorption disappeared, but absorption peaks of epoxy group appeared at 790 cm⁻¹ and 810 cm⁻¹ in the IR spectrum of the product. The IR spectrum measurement was performed using a JASCO FT/IR-4200 type A Fourier transform infrared spectrophotometer (FTIR) and using a TGS detector at a resolution of 4 cm⁻¹ with a number of scans of 16.

Based on the analysis results, the product prepared in Example 1 was identified to have a structure of Formula (1-1) :

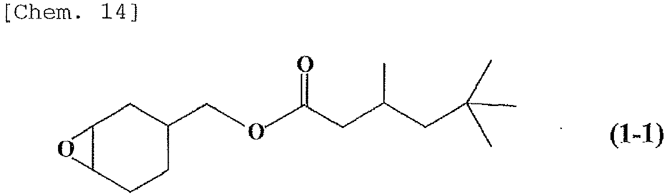

### Volatility Evaluation

The 3,4-epoxycyclohexylmethyl 3,5,5-trimethylhexanoate prepared in Example 1 was subjected to boiling point measurement under reduced pressure using a distillator. As a result, this product was found to have boiling points of 191°C, 218°C, and 233°C respectively at 11 Torr (1467 Pa), 31 Torr (4133 Pa), and 50 Torr (6666 Pa).

The measured boiling points at the individual pressures were plotted on a logarithmic graph (semilogarithmic graph with the logarithmic scale indicating the pressure) to give an approximate curve. Using the approximate curve, a boiling point of the product at normal atmospheric pressure (760 Torr; 101325 Pa) was calculated and found to be 307°C. As is demonstrated above, the 3,4-epoxycyclohexylmethyl 3,5,5-trimethylhexanoate prepared in Example 1 had a sufficiently high boiling point.

### Evaluation of Reactivity with Acid

A mixture was prepared by blending 5.2 g of the 3,4-epoxycyclohexylmethyl 3,5,5-trimethylhexanoate prepared in Example 1 (product of Example 1), 1.4 g of 2-ethylhexanoic acid (trade name Octanoic Acid, supplied by Kyowa Hakko Chemical Co., Ltd.), and 8.5 g of 1,3,5-trimethylbenzene (reagent, supplied by Wako Pure Chemical Industries, Ltd.). The mixture was stirred at 150°C for 6 hours. The acid value and oxirane oxygen content of the mixture were measured before the stirring with heating, and 2 hours, 4 hours, and 6 hours into the stirring.

The oxirane oxygen content measurement in the evaluation of reactivity with acid was performed by HBr titrimetry in which titration was performed with a N/10 hydrogen bromide solution in acetic acid and with crystal violet as an indicator. The acid value measurement was performed in conformance with a method described in JIS K 0070.

Instead of 3,4-epoxycyclohexylmethyl 3,5,5-trimethylhexanoate, there were prepared 3,4-epoxycyclohexylmethyl (3,4-epoxy)cyclohexane carboxylate (trade name CELLOXIDE 2021P, supplied by Daicel Corporation), 4-vinylcyclohexene oxide (trade name CELLOXIDE 2000, supplied by Daicel Corporation), 1-ethyl-4-(2-methyloxiranyl)-7-oxabicyclo[4.1.0]heptane (trade name CELLOXIDE 3000, supplied by Daicel Corporation), D-limonene oxide (supplied by Nippon Terpene Chemicals, Inc.), and di-2-ethylhexyl epoxyhexahydrophthalate (trade name SANSO CIZER E-PS, supplied by New Japan Chemical Co., Ltd.). These epoxides were used in charged amounts given in Table 1 and subjected to evaluation of reactivity with an acid (2-ethylhexanoic acid) by the above procedure.

Table 2 gives the oxirane oxygen contents and acid values measured in the evaluation. Table 2 also gives acid residual percentages as calculated from the acid values. The acid residual percentages are percentages of the acid present in the system (in the mixture) at the individual timings (after 2 hours, 4 hours, and 6 hours) in which the amount of the acid present in the system before the stirring with heating (0 hour) was defined as 100%.

[Table 1]

**TABLE 1**

| Epoxide type | Charged amount (g) | | |
|---|---|---|---|
| | Epoxide | 2-Ethylhexanoic acid | 1,3,5-Trimethylbenzene |
| Product of Example 1 | 5.2 | 1.4 | 8.5 |
| CELLOXIDE 2021P | 2.4 | 1.4 | 11.2 |
| CELLOXIDE 2000 | 2.4 | 1.4 | 11.2 |
| CELLOXIDE 3000 | 1.6 | 1.4 | 12.0 |
| D-Limonene oxide | 2.9 | 1.4 | 10.7 |
| SANSO CIZER E-PS | 7.9 | 1.4 | 5.7 |

[Table 2]

**TABLE 2**

| Epoxide type | | Stirring time (150°C) | | | |
|---|---|---|---|---|---|
| | | 0 hr (before heating) | 2 hrs | 4 hrs | 6 hrs |
| Product of Example 1 | Oxirane oxygen content [%] | 1.82 | 1.20 | 1.00 | 0.89 |
| | Acid value [KOH-mg/g] | 37.66 | 20.32 | 13.13 | 9.98 |
| | Acid residual percentage [%] | 100 | 54 | 35 | 27 |
| CELLOXIDE 2021P | Oxirane oxygen content [%] | 2.00 | 1.51 | 1.33 | 1.11 |
| | Acid value [KOH-mg/g] | 37.56 | 21.23 | 14.54 | 11.22 |
| | Acid residual percentage [%] | 100 | 57 | 39 | 30 |
| CELLOXIDE 2000 | Oxirane oxygen content [%] | 1.92 | 1.46 | 1.25 | 1.15 |
| | Acid value [KOH-mg/g] | 35.90 | 23.80 | 17.54 | 12.90 |
| | Acid residual percentage [%] | 100 | 66 | 49 | 36 |
| CELLOXIDE 3000 | Oxirane oxygen content [%] | 1.81 | 0.91 | 0.71 | 0.61 |
| | Acid value [KOH-mg/g] | 36.04 | 24.52 | 22.04 | 18.65 |
| | Acid residual percentage [%] | 100 | 68 | 61 | 52 |
| D-Limonene oxide | Oxirane oxygen content [%] | 1.92 | 1.56 | 1.38 | 1.25 |
| | Acid value [KOH-mg/g] | 38.36 | 26.27 | 22.44 | 19.15 |
| | Acid residual percentage [%] | 100 | 68 | 58 | 50 |
| SANSO CIZER E-PS | Oxirane oxygen content [%] | 1.84 | 1.72 | 1.63 | 1.53 |
| | Acid value [KOH-mg/g] | 38.13 | 33.80 | 32.16 | 27.97 |
| | Acid residual percentage [%] | 100 | 89 | 84 | 73 |

A comparison of acid residual percentage in the evaluation results given in Table 2 demonstrated that the cycloaliphatic epoxide according to the present invention (product of Example 1; 3,4-epoxycyclohexylmethyl 3,5,5-trimethylhexanoate) had higher acid-scavenging capability (i.e., higher reactivity with the acid) as compared with the other epoxides.

### Industrial Applicability

The cycloaliphatic epoxide according to the present invention is preferably usable particularly as a variety of additives such as reactive diluents and stabilizers (acid scavengers). In addition, the cycloaliphatic epoxide according to the present invention is also usable in other various uses such as optical semiconductor encapsulants, adhesives, electrical insulating materials, laminated sheets, coatings, inks, coating materials, sealants, resists, composite materials, transparent substrates, transparent sheets, transparent films, optical devices, optical lenses, optical elements, stereolithographic materials, electronic papers, touch-screen panels, solar cell substrates, optical waveguides, light guide plates, and holographic memories.

## Claims

1. A cycloaliphatic epoxide represented by Formula (1): 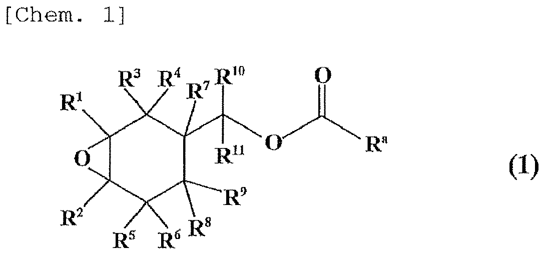 wherein R¹ to R¹¹ are each independently selected from hydrogen and C₁-C₄ straight or branched chain alkyl; and R^{a} represents C₈-C₂₃ branched chain alkyl.

2. The cycloaliphatic epoxide according to claim 1,
wherein R^{a} is C₈-C₁₇ branched chain alkyl.

3. The cycloaliphatic epoxide according to one of claims 1 and 2,
wherein R¹ to R¹¹ are each hydrogen.

4. A method for producing a cycloaliphatic epoxide, the method comprising the step of
A) allowing a compound represented by Formula (2) to react with an oxidant to form a cycloaliphatic epoxide represented by Formula (1) as the cycloaliphatic epoxide according to any one of claims 1 to 3, Formulae (2) and (1) being expressed as follows: 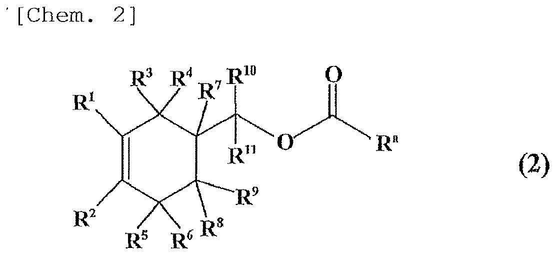
wherein R¹ to R¹¹ are each independently selected from hydrogen and C₁-C₄ straight or branched chain alkyl; and R^{a} represents C₈-C₂₃ branched chain alkyl, 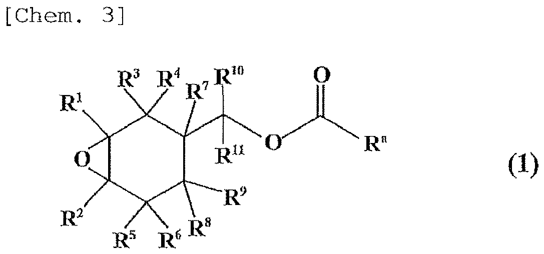 wherein R¹ to R¹¹ and R^{a} are as defined above.

5. The method for producing a cycloaliphatic epoxide according to claim 4, further comprising, before the step A), the step of
B) allowing a compound represented by Formula (3) to react with a compound represented by Formula (4) or a derivative thereof to form the compound represented by Formula (2), Formulae (3) and (4) being expressed as follows: 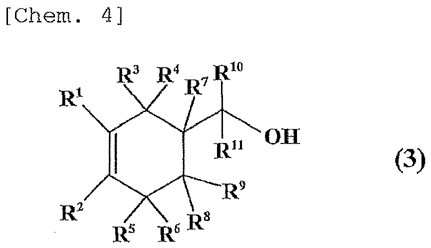
wherein R¹ to R¹¹ are as defined above, 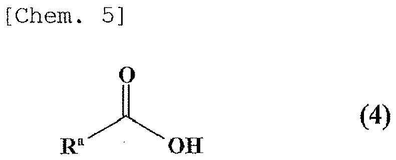 wherein R^{a} is as defined above.
